(19) Europäisches Patentamt
European Patent Office
Office européen des brevets

(11) Veröffentlichungsnummer : **0 021 345**
**B1**

(12) # EUROPÄISCHE PATENTSCHRIFT

(45) Veröffentlichungstag der Patentschrift : **11.05.83**

(51) Int. Cl.³ : **C 07 D249/08**, A 01 N 43/64

(21) Anmeldenummer : **80103414.1**

(22) Anmeldetag : **19.06.80**

(54) **Alpha-Azolylglykolderivate, ihre Herstellung, sie enthaltende pflanzenwachstumsregulierende Mittel und ihre Verwendung.**

(30) Priorität : **29.06.79 DE 2926280**

(43) Veröffentlichungstag der Anmeldung : **07.01.81 Patentblatt 81/01**

(45) Bekanntmachung des Hinweises auf die Patenterteilung : **11.05.83 Patentblatt 83/19**

(84) Benannte Vertragsstaaten : **AT BE CH DE FR GB IT LI NL SE**

(56) Entgegenhaltungen :
**DE A 2 105 490**
**DE A 2 600 799**

**Die Akte enthält technische Angaben, die nach dem Eingang der Anmeldung eingereicht wurden und die nicht in dieser Patentschrift enthalten sind.**

(73) Patentinhaber : **BASF Aktiengesellschaft**
**Carl-Bosch-Strasse 38**
**D-6700 Ludwigshafen (DE)**

(72) Erfinder : **Zeeh, Bernd, Dr.**
**Thorwaldsenstrasse 5**
**D-6700 Ludwigshafen (DE)**
Erfinder : **Goetz, Norbert, Dr.**
**Schoefferstrasse 25**
**D-6520 Worms (DE)**
Erfinder : **Ammermann. Eberhard, Dr.**
**Sachsenstrasse 3**
**D-6700 Ludwigshafen (DE)**
Erfinder : **Pommer, Ernst-Heinrich, Dr.**
**Berliner Platz 7**
**D-6703 Limburgerhof (DE)**

**EP 0 021 345 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

α-Azolylglykolderivate, ihre Herstellung, sie enthaltende pflanzenwachstumsregulierende Mittel und ihre Verwendung

Die vorliegende Erfindung betrifft neue α-Azolylglykolderivate, Verfahren zu deren Herstellung, wachstumsregulierende Mittel, die diese Wirkstoffe enthalten, sowie Verfahren zur Regulierung des Pflanzenwachstums mit diesen Mitteln und deren Verwendung für diese Zwecke.

Es ist bekannt, daß N,O-Acetale mit einem O-Alkylrest wie zum Beispiel der 1-(1',2',4'-Triazol-1'-yl)-butyl-1-ethyl-ether (DE-OS 26 40 823, Beispiel 4) fungizide Wirksamkeit haben. Ferner sind fungizide Ether und Ester bekannt wie beispielsweise 1-(2'-(2",4"-Dichlorphenyl-)-2'-(2"-propenyloxy-)-ethyl)-1H-imidazol (DE-OS 20 63 857) oder 2-Acetoxy-1-(4-chlorphenoxy)-1-(1',2',4'-triazol-1'-yl)-3,3-dimethylbutan (DE-OS 26 00 799). Schließlich sind in der DE-OS 21 05 490 antimykotisch wirksame Imidazolderivate beschrieben.

Es wurden nun Triazolderivate mit pflanzenwachstumsregulierender Wirkung gefunden.

Gegenstand der Erfindung sind α-Azolylglykolderivate der allgemeinen Formel I

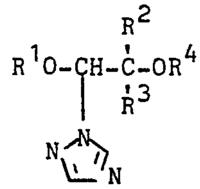

(I)

in welcher

$R^1$ $C_{1-4}$-Alkyl

$R^2$ $C_{1-6}$-Alkyl oder gegebenenfalls durch Halogen substituiertes Phenyl,

$R^3$ Wasserstoff oder $C_{1-6}$-Alkyl,

$R^4$ Alkyl oder Alkenyl mit bis zu 10 C-Atomen oder halogensubstituiertes Benzyl

bedeuten, sowie deren für Pflanzen verträgliche Salze und Metallkomplexe.

In der Formel I steht $R^1$ beispielsweise für einen Methyl-, Ethyl-, n-Propyl-, iso-Propyl-, n-Butyl-, iso-Butyl- oder tert.-Butylrest.

$R^2$ steht für einen Alkylrest mit 1 bis 6 Kohlenstoffatomen, der unverzweigt sein kann wie beispielsweise ein Methyl, Ethyl, Propyl, Butyl oder Pentylrest oder verzweigt sein kann und 3 bis 6 Kohlenstoffatome umfassen kann wie beispielsweise ein Isopropyl, tert.-Butyl oder ein 3-Methyl-butyl-1-rest. Ferner steht $R^2$ für einen unsubstituierten oder durch Halogen, vorzugsweise Chlor, mono- oder disubstituierten Phenylrest.

$R^3$ steht für Wasserstoff, für einen unverzweigten Alkylrest mit 1 bis 6 Kohlenstoffatomen wie beispielsweise für einen Methyl-, Ethyl-, Propyl-, Butyl- oder Pentylrest oder für einen verzweigten Alkylrest mit 3 bis 6 Kohlenstoffatomen — wie für einen Isopropyl- oder iso-Butylrest —.

$R^4$ steht für einen Alkylrest mit bis zu 10 Kohlenstoffatomen, der beispielsweise unverzweigt — wie ein Methyl-, Ethyl-, n-Propyl-, n-Pentyl- oder ein n-Decylrest — oder verzweigt — wie beispielsweise ein Isopropyl-, 2-Methyl-propyl-1- oder 3,3-Dimethyl-n-butyl-1-rest — sein kann, ferner für einen durch Fluor, Chlor, Brom oder Iod substituierten Benzylrest.

Die neuen α-Azolylglykolderivate besitzen im Acetal-C-atom und — falls $R^2$ und $R^3$ verschieden sind — im Carbinol-C-atom je ein Asymmetriezentrum. Je nach der Beschaffenheit von $R^4$ kommen eventuell weitere Asymmetriezentren dazu. Mit üblichen Trennmethoden können die Verbindungen in Form einheitlicher Enantiomerer bzw. Diastereomerer erhalten werden. In der Praxis kann man sowohl die einheitlichen Enantiomeren bzw. Diastereomeren wie auch die bei der Synthese üblicherweise anfallenden Gemische verwenden. Letztere werden bevorzugt verwendet.

Die neuen α-Azolylglykolderivate der Formel I lassen sich herstellen indem man

a) Alkohole der Formel II

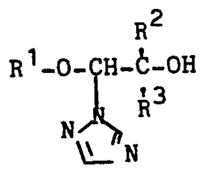

(II)

in welcher $R^1$, $R^2$, $R^3$ und X die oben angegebenen Bedeutungen haben oder ihre Alkali- oder quartären Ammoniumsalze mit einer Verbindung der Formel III

$$R^4—L \qquad (III)$$

worin $R^4$ die oben angegebenen Bedeutungen hat und L für eine nucleophil verdrängbare Abgangsgruppe steht, gegebenenfalls in Gegenwart eines Lösungs- oder Verdünnungsmittels und/oder anorganischer oder organischer Basen sowie gegebenenfalls unter Zusatz eines Reaktionsbeschleunigers bei Temperaturen zwischen 0 und 120 °C umsetzt oder

b) Acetale der Formel IV

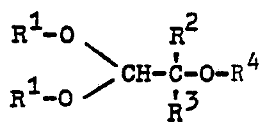

(IV)

in welcher $R^1$, $R^2$, $R^3$ und $R^4$ die oben erläuterten Bedeutungen haben, zunächst mit Acetylchlorid oder -bromid und anschließend mit 1,2,4-Triazol oder dessen Alkali- oder Erdalkalisalzen gegebenenfalls in Gegenwart eines Lösungs- oder Verdünnungsmittels und/oder anorganischer oder organischer Basen bei Temperaturen zwischen 0 und 100 °C umsetzt.

Die für das Verfahren a) als Ausgangsmaterial verwendeten Alkohole der Formel II lassen sich z. B. nach folgenden Methoden herstellen :

1. Ein Keton der Formel V

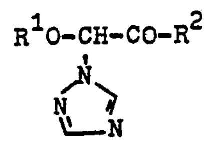

(V)

in der $R^1$ und $R^2$ die oben erläuterten Bedeutungen haben, wird katalytisch oder mit komplexen Hydriden in Gegenwart eines Lösungsmittels und gegebenenfalls in Gegenwart eines Reaktionsbeschleunigers bei Temperaturen zwischen 0 und 100 °C reduziert.

Die Verbindungen V sind herstellbar durch Umsetzung von α-Halogenethern der Formel VI

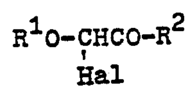

(VI)

in der $R^1$ und $R^2$ die oben erwähnte Bedeutung haben und Hal Chlor oder Brom bedeutet, mit 1,2,4-Triazol oder dessen Alkali- oder Erdalkalisalzen in Gegenwart eines Lösungsmittels und einer Base bei Temperaturen zwischen 0 und 100 °C.

Die α-Halogenether der Formel VI können nach bekannten Methoden hergestellt werden (vgl. DE-OS 22 01 063 und B. Mylo, Chem. Ber. *44* (1911) S. 3212, Straus und Weber Ann. *498* (1932) S. 124).

2. Ein Keton der Formel V wird mit Grignardreagentien der Formel VII

$$R^3MgBr \qquad (VII)$$

in der $R^3$ die oben erläuterte Bedeutung hat, in Gegenwart inerter Lösungsmittel bei Temperaturen zwischen 0 und 80 °C umgesetzt.

3. Ein Acetal der Formel VIII

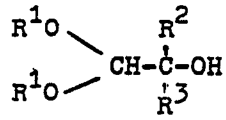

(VIII)

in der $R^1$, $R^2$ und $R^3$ die oben angegebenen Bedeutungen haben, wird mit Acetylchlorid oder -bromid und anschließend mit 1,2,4-Triazol in Gegenwart eines Lösungsmittels und einer Base bei Temperaturen zwischen 0 und 100 °C umgesetzt.

Die Verbindungen der Formel III sind bekannt. In der Formel III bedeutet L beispielsweise : Halogen — wie Chlor, Brom oder Iod —, Alkylsulfat, gegebenenfalls substituierte Alkyl-sulfonyloxyreste — wie Methansulfonyloxy- oder Trifluor- methansulfonyloxyreste —, oder Arylsulfonyloxyreste — wie der Tosylatrest —. Ferner seien als nucleophil verdrängbare Abgangsgruppen besonders für die reaktiven Säurederivate der Formel III zusätzlich Azole — wie Imidazol oder Triazol — und Acyloxygruppen wie in Säureanhydriden genannt.

Geeignete anorganische oder organische Basen, die gegebenenfalls auch als säurebindende Mittel in die Verfahren a) oder b) eingesetzt werden können, sind beispielsweise Alkali- und Erdalkalihydroxide — wie Natriumhydroxid, Kaliumhydroxid, Calciumhydroxid —, Alkalicarbonate — wie Kalium- oder Natriumcarbonat —, Alkalihydride — wie Natriumhydrid —, Alkali- oder Erdalkalialkoholate — wie Natriummethylat, Magnesiummethylat oder Natriumisopropylat —, oder tertiäre Amine — wie Trimethylamin, Triethylamin, N,N-Dimethylanilin, N,N-Dimethylcyclohexylamin, N-Methylpiperidin oder Pyridin —. Es können aber auch andere übliche Basen verwendet werden.

Mit geeigneten Basen wie zum Beispiel Alkalihydrid — wie Natriumhydrid- oder Lithiumalkyl — z. B. Butyllithium- oder mit Alkali- oder Erdalkalialkoholaten — wie Natriummethylat — können die Alkohole der Formel II auch zunächst in ihre Alkoholat-Salze überführt werden und dann als solche zur Reaktion gebracht werden.

Zu den bevorzugten Lösungs- bzw. Verdünnungsmitteln gehören Halogenkohlenwasserstoffe — wie Methylenchlorid, Chloroform, 1,2-Dichlorethan, Chlorbenzol —, aliphatische oder aromatische Kohlenwasserstoffe — wie Cyclohexan, Petrolether, Benzol, Toluol oder Xylole —, Ester — wie Essigsäureethylester —, Amide — wie Dimethylformamid —, Nitrile — wie Acetonitril —, Sulfoxide — wie Dimethylsulfoxid —, Ketone — wie Aceton oder Methylethylketon —, Ether — wie Diethylether, Tetrahydrofuran oder Dioxan —, oder entsprechende Gemische.

Als Reaktionsbeschleuniger kommen vorzugsweise Metallhalogenide — wie Kaliumiodid —, Kronenether, quartäre Ammoniumverbindungen — wie Tetrabutylammoniumiodid — oder Säuren oder Kombinationen dieser Reaktionsbeschleuniger in Frage.

Die im Verfahren b) als Ausgangsmaterialien eingesetzten Acetale der Formel IV können nach bekannten Methoden durch Umsetzung der Alkohole der Formel IX

$$(R^1O)_2CH-\overset{\displaystyle R^2}{\underset{\displaystyle R^3}{\overset{|}{\underset{|}{C}}}}-OH \qquad \text{(IX)}$$

mit Verbindungen der Formel III hergestellt werden.

Die Alkohole der Formel IX sind teilweise bekannt wie zum Beispiel das 1,1-Dimethoxy-2-methylbutin-3-ol-2 (DE-PS 17 68 877), oder das 1,1-Dimethoxy-2-methylbuten-3-ol-2 (DE-PS 11 15 238). Sie können aber auch nach bekannten Methoden hergestellt werden, indem man Ketone der Formel X

$$(R^1O)_2CH—CO—R^2 \qquad \text{(X)}$$

katalytisch oder mit komplexen Hydriden hydriert oder mit Grignardreagentien der Formel VII umsetzt.

Zur Herstellung der erfindungsgemäßen α-Azolylglykol-derivate der Formel I nach Verfahren b) verwendet man bei der Umsetzung der Acetale der Formel IV mit Acetylchlorid oder Acetylbromid zweckmäßig kein oder ein inertes Lösungs- oder Verdünnungsmittel wie Ether, Methylenchlorid, Chloroform, Cyclohexan oder Toluol. Die Reaktionstemperatur kann zwischen 20 und 100 °C liegen und richtet sich nach dem Rest $OR^1$.

Für die Reaktion des intermediär entstehenden α-Halogenethers mit 1,2,4-Triazol verwendet man vorteilhaft Lösungs- oder Verdünnungsmittel wie beispielsweise Methylenchlorid, Chloroform, 1,2-Dichlorethan, Chlorbenzol, aliphatische oder aromatische Kohlenwasserstoffe — wie Cyclohexan, Petrolether, Benzol, Toluol oder Xylole —, Ester — wie Essigsäureethylester —, Amide — wie Dimethylformamid-, Nitrile — wie Acetonitril —, Sulfoxide — wie Dimethylsulfoxid —, Ketone — wie Aceton oder Methylethylketon —, Ether — wie Diethylether, Tetrahydrofuran oder Dioxan — oder entsprechende Gemische.

Geeignete anorganische oder organische Basen, die gegebenenfalls auch als säurebindende Mittel in das Verfahren b) eingesetzt werden können, sind beispielsweise Alkali- und Erdalkalihydroxide — wie Natriumhydroxid, Kaliumhydroxid, Calciumhydroxid —, Alkalicarbonate — wie Kalium- oder Natriumcarbonat —, Alkalihydride — wie Natriumhydrid —, Alkali- oder Erdalkalialkoholate — wie Natriummethylat, Magnesiummethylat oder Natriumisopropylat —, tertiäre Amine — wie Trimethylamin, Triethylamin, N,N-Dimethylanilin, N,N-Dimethylcyclohexylamin, N-Methylpiperidin oder Pyridin — oder Azole — wie 1,2,

4-Triazol oder Imidazol —. Es können aber auch andere übliche Basen verwendet werden.

Mit geeigneten Basen wie zum Beispiel Alkalihydrid — wie Natriumhydrid —, Lithiumalkyl — wie Butyllithium —, oder Alkali- oder Erdalkalialkoholaten — wie Natriummethylat — kann 1,2,4-Triazol auch in einer vorgeschalteten Umsetzung zunächst das Salz überführt werden und dann als solche zur Reaktion gebracht werden.

Die erfindungsgemäßen Umsetzungen werden im allgemeinen bei Temperaturen zwischen 0 und 150 °C in einem Zeitraum von 1 bis 60 Stunden durchgeführt, drucklos oder unter Druck, kontinuierlich oder diskontinuierlich.

Zur Isolierung der erfindungsgemäßen Verbindungen wird nach üblichen Methoden vorgegangen. Im allgemeinen bedürfen die anfallenden Produkte keiner weiteren Reinigung, sie können aber auch nach bekannten Methoden wie Umkristallisation, Extraktion, Destillation oder Chromatographie weiter gereinigt werden.

Falls gewünscht, werden die α-Azolylglykolderivate der Formel I anschließend nach bekannten Verfahren in ihre für Pflanzen verträglichen Salze oder in ihre Metallkomplexe überführt.

Zur Salzbildung eignen sich beispielsweise : Salzsäure, Bromwasserstoffsäure, Schwefelsäure, Salpetersäure, Phosphorsäure, Essigsäure oder Dodecylbenzolsulfonsäure. Die Wirksamkeit der Salze geht auf das Kation zurück, so daß die Wahl des Anions beliebig ist.

Metallkomplexe bilden sich durch Anlagerung der neuen Verbindungen an die Kationen von Metallsalzen. Besonders eignen sich hierfür Kupfer(II)-chlorid, Kupfer(II)-sulfat, Kupfer(II)-nitrat, Zink(II)-chlorid, Eisen(III)-chlorid, Mangan(II)-chlorid, Nickel(II)-bromid.

Die folgenden Beispiele schildern die Herstellung der neuen Substanzen

## Herstellung der Ausgangsstoffe

a) Verfahren 1

Zu 48 g 1,1-Dimethoxy-3,3-dimethylbutan-2-on (vgl. J.B. Wright J. Am. Chem. Soc. *77* (1955) S. 4 883) werden unter Rühren 36,8 g Acetylbromid getropft. Dabei steigt die Temperatur auf 53 °C an. Nach einstündigem Rühren wird diese Lösung zu einer Lösung von 41,4 g Triazol in 100 ml Dimethylformamid und 100 ml Tetrahydrofuran getropft. Es wird drei Stunden gerührt, dann das Reaktionsgemisch eingeengt, in Methylenchlorid aufgenommen und dreimal mit je 50 ml Wasser gewaschen. Die organische Phase wird abgetrennt, getrocknet und eingeengt. Das verbleibende Öl wird über eine Kolonne destilliert. Zwischen 84 und 86 °C/0,1 mbar gehen 44 g 1-(1',2',4'-Triazol-1'-yl-)-1-methoxy-3,3-dimethylbutan-2-on über.

39,4 g der so erhaltenen Verbindung werden in 80 ml Methanol bei 10-20 °C portionsweise mit 4,5 g Natriumborhydrid versetzt. Anschließend wird 1 Stunde bei Rückflußtemperatur gerührt. Das Reaktionsgemisch wird in 80 ml Wasser eingerührt und dreimal mit je 100 ml Methylenchlorid extrahiert. Die organische Phase wird abgetrennt, getrocknet und eingeengt. Das verbleibende Öl kristallisiert aus Petrolether aus. Man erhält so 34 g 1-(1',2',4'-Triazol-1'-yl)-1-methoxy-3,3-dimethylbutanol-2 vom Schmelzpunkt 62-64 °C.

| | C | H | N |
|---|---|---|---|
| ber. | 54,3 | 8,6 | 21,1 |
| gef. | 54,5 | 8,4 | 21,2 |

b) Verfahren 2

Zu einer Lösung von 0,2 Mol 4-Chlorphenylmagnesiumbromid (hergestellt auf 4,9 g Magnesium und 38,3 g 4-Bromchlorbenzol) in 150 ml Ether wird eine Lösung von 1-(1',2',4'-Triazol-1'-yl)-1-methoxy-aceton in 100 ml Ether getropft. Anschließend wird 5 Stunden bei Rückflußtemperatur gerührt. Zu dem abgekühlten Reaktionsgemisch werden 50 g Eis und dann 25 %ige wäßrige Ammoniumchloridlösung zugetropft, bis sich die Phasen klar trennen. Die organische Phase wird abgetrennt und die wäßrige Phase zweimal mit je 100 ml Ether extrahiert. Die vereinigten Etherphasen werden mit Wasser neutral gewaschen, getrocknet und eingeengt. Aus Petrolether erhält man 23 g kristallines 1-(1',2',4'-Triazol-1'-yl)-1-methoxy-2-(4'-chlorphenyl)-propanol-2 vom Schmelzpunkt 80-82 °C.

c) Verfahren 3

Zu 14,8 g 1,1-(Dimethoxy-)-2-methyl-butanol-2 werden unter Rühren 12,3 g Acetylbromid getropft. Dabei steigt die Temperatur bis 60 °C an. Nach einer Stunde Rühren wird dieses Reaktionsgemisch zu einer Lösung von 13,8 g Triazol in 100 ml Tetrahydrofuran und 50 ml Dimethylformamid getropft. Nach dem Rühren über Nacht wird das Reaktionsgemisch eingeengt, in Methylenchlorid aufgenommen und dreimal mit je 50 ml Wasser gewaschen. Die organische Phase wird getrocknet und eingeengt. Das verbleibende Öl wird destilliert. Bei 95-110 °C/0,4 mbar gehen 6 g 1-(1',2',4'-Triazol-1'-yl)-1-methoxy-2-methylbutanol-2 über.

Analog a) bis c) können alle Alkohole der Formel II hergestellt werden.

## Herstellung der Endprodukte

## Beispiel 1 (Verfahren a)

Zu einer Suspension von 2 g Natriumhydrid in 80 ml Tetrahydrofuran wird bei Raumtemperatur eine Lösung von 13,9 g 1-(1',2',4'-Triazol-1'-yl)-1-methoxy-3,3-dimethylbutan-2-ol in 30 ml Tetrahydrofuran zugetropft. Nach dreistündigem Rühren werden 15 g p-Chlorbenzylbromid zugetropft. Anschließend wird über Nacht gerührt. Nach der Zugabe von 100 ml Wasser wird die organische Phase abgetrennt und die wäßrige Phase noch zweimal ausgeethert. Die vereinigten Etherphasen werden getrocknet und eingeengt. Das zurückbleibende Öl wird destilliert. Bei 156-158 °C und 0,04 mbar gehen 9,3 g 1-(1',2',4'-Triazol-1'-yl)-1-methoxy-3,3-dimethylbutyl-2-p-Chlorbenzylether über.

## Beispiel 2 (Verfahren b)

Zu 17,6 g 1,1-Dimethoxy-3,3-dimethyl-butyl-2-methylether werden 12,3 g Acetylbromid getropft, wobei die Temperatur auf 55 °C ansteigt. Nach einstündigem Rühren wird diese Lösung zu einer Lösung von 13,8 g Triazol in 50 ml Dimethylformamid und 50 ml Tetrahydrofuran getropft. Nach drei Stunden wird das Reaktionsgemisch eingeengt, in 200 ml Methylenchlorid aufgenommen und dreimal mit je 50 ml Wasser ausgeschüttelt. Die organische Phase wird abgetrennt, getrocknet und eingeengt. Das zurückbleibende Öl wird destilliert. Bei 75-77 °C und 0,3 mbar gehen 7,4 g 1-(1', 2', 4'-Triazol-1'-yl)-1-methoxy-3,3-dimethylbutyl-2-methylether über.

| | C | H | N |
|---|---|---|---|
| ber. | 56,3 | 9,0 | 19,7 |
| gef. | 56,6 | 9,0 | 20,1 |

Analog können folgende α-Azolylglyokolderivate der Formel I hergestellt werden.

Tabelle 1

| Nr. | $R^1$ | $R^2$ | $R^3$ | $R^4$ | Kp °C/mbar |
|---|---|---|---|---|---|
| 3 | $CH_3$ | $CH_2-CH_2-CH(CH_3)_2$ | H | $CH_3$ | 103 - 105/0,7 |
| 4 | $CH_3$ | $CH_3$ | $C_2H_5$ | $CH_3$ | 88 - 90/0,5 |
| 5 | $CH_3$ | $C_6H_5$ | $CH_3$ | $CH_3$ | Öl |
| 6 | $CH_3$ | $4-Cl-C_6H_4$ | $CH_3$ | $CH_3$ | Fp 112 - 114 |
| 7 | $CH_3$ | $4-Cl-C_6H_4$ | $CH_3$ | $CH_2CH=CH_2$ | Fp 78 - 80 |
| 8 | $CH_3$ | $4-Cl-C_6H_4$ | $CH_3$ | $CH_2-p-Cl-C_6H_4$ | Fp 148 - 149 |
| 9 | $CH_3$ | tert-$C_4H_9$ | H | $CH_2-CH=CH_2$ | 82 - 85/0,1 |
| 10 | $CH_3$ | tert-$C_4H_9$ | H | $CH_2-2,4-Cl_2-C_6H_3$ | Öl |
| 11 | $CH_3$ | tert-$C_4H_9$ | $CH_3$ | $CH_3$ | 82 - 86/0,1 |
| 12 | $CH_3$ | tert-$C_4H_9$ | $CH_3$ | $CH_2-CH=CH_2$ | 98 - 100/0,3 |
| 13 | $CH_3$ | tert-$C_4H_9$ | $CH_3$ | $CH_2-p-Cl-C_6H_4$ | Fp 73 - 75 |
| 14 | $CH_3$ | $CH_3$ | H | $CH_3$ | 74 - 75/0,2 |
| 15 | $CH_3$ | $CH_3$ | H | $C_2H_5$ | |
| 16 | $CH_3$ | $CH_3$ | H | $CH_2CH=CH_2$ | 82 - 85/0,2 |
| 17 | $CH_3$ | $CH_3$ | H | $CH_2-p-Cl-C_6H_4$ | Öl |
| 18 | n-$C_4H_9$ | tert-$C_4H_9$ | H | $CH_2-p-Cl-C_6H_4$ | Öl |
| 19 | n-$C_4H_9$ | tert-$C_4H_9$ | $CH_3$ | $CH_2-p-Cl-C_6H_4$ | Öl |
| 20 | $CH_3$ | $2,4-Cl_2-C_6H_3$ | H | $CH_3$ | Fp 92 - 94 |
| 21 | $CH_3$ | $2,4-Cl_2-C_6H_3$ | H | $CH_2-CH=CH_2$ | Öl |
| 22 | $CH_3$ | $2,4-Cl_2-C_6H_3$ | H | $CH_2-p-Cl-C_6H_4$ | Fp 95 - 110 |
| 23 | $C_2H_5$ | $2,4-Cl_2-C_6H_3$ | H | $CH_3$ | Fp 85 - 87 |
| 24 | $C_2H_5$ | $2,4-Cl_2-C_6H_3$ | H | $CH_2-CH=CH_2$ | Öl |
| 25 | $CH_3$ | $C_6H_5$ | $CH_3$ | $CH_2-CH=CH_2$ | Öl |
| 26 | $CH_3$ | $C_6H_5$ | $CH_3$ | $CH_2-p-Cl-C_6H_4$ | Fp 128 - 130 |
| 27 | n-$C_4H_9$ | $4-Cl-C_6H_4$ | $CH_3$ | $CH_3$ | 143 - 149/0,0015 |
| 28 | n-$C_4H_9$ | $4-Cl-C_6H_4$ | $CH_3$ | $CH_2-CH=CH_2$ | 147 - 152/0,0015 |

Tabelle 1 (Forts.)

| Nr. | $R^1$ | $R^2$ | $R^3$ | $R^4$ | Kp °C/mbar |
|---|---|---|---|---|---|
| 29 | $n-C_4H_9$ | $4-Cl-C_6H_4$ | $CH_3$ | $CH_2-p-Cl-C_6H_4$ | 205 - 218/0,015 |
| 30 | $iso-C_4H_9$ | $4-Cl-C_6H_4$ | $CH_3$ | $CH_3$ | 142 - 148/0,0015 |
| 31 | $iso-C_4H_9$ | $4-Cl-C_6H_4$ | $CH_3$ | $C_2H_5$ | 151 - 156/0,0015 |
| 32 | $CH_3$ | $2,4-Cl_2-C_6H_3$ | $CH_3$ | $CH_3$ | Fp 98 - 94 |
| 33 | $CH_3$ | $2,4-Cl_2-C_6H_3$ | $CH_3$ | $CH_2-CH=CH_2$ | Öl |
| 34 | $C_2H_5$ | $2,4-Cl_2-C_6H_3$ | $CH_3$ | $CH_3$ | Fp 57 - 59 |
| 35 | $C_2H_5$ | $2,4-Cl_2-C_6H_3$ | $CH_3$ | $CH_2-CH=CH_2$ | Fp 79 - 80 |
| 36 | $tert-C_4H_9$ | $CH_3$ | H | $CH_3$ | |
| 37 | $tert-C_4H_9$ | $CH_3$ | H | $CH_2-CH=CH_2$ | |
| 38 | $tert-C_4H_9$ | $CH_3$ | H | $CH_2-p-Cl-C_6H_4$ | |
| 39 | $tert-C_4H_9$ | $CH_3$ | $CH_3$ | $CH_2-p-Cl-C_6H_4$ | |
| 40 | $tert-C_4H_9$ | $C_6H_5$ | $CH_3$ | $CH_3$ | |
| 41 | $tert-C_4H_9$ | $C_6H_5$ | $CH_3$ | $CH_2-CH=CH_2$ | |
| 42 | $tert-C_4H_9$ | $C_6H_5$ | $CH_3$ | $CH_2-p-Cl-C_6H_4$ | |
| 43 | $tert-C_4H_9$ | $4-Cl-C_6H_4$ | $CH_3$ | $CH_3$ | |
| 44 | $tert-C_4H_9$ | $4-Cl-C_6H_4$ | $CH_3$ | $CH_2-CH=CH_2$ | |
| 45 | $tert-C_4H_9$ | $4-Cl-C_6H_4$ | $CH_3$ | $CH_2-p-Cl-C_6H_4$ | |
| 46 | $tert-C_4H_9$ | $tert-C_4H_9$ | $CH_3$ | $CH_3$ | |
| 47 | $tert-C_4H_9$ | $tert-C_4H_9$ | $CH_3$ | $CH_2-CH=CH_2$ | |
| 48 | $tert-C_4H_9$ | $tert-C_4H_9$ | $CH_3$ | $CH_2-p-Cl-C_6H_4$ | |

Die erfindungsgemäßen Verbindungen und ihre Salze und Metallkomplexverbindungen stellen gute Pflanzenbehandlungsmittel dar. Sie können als Mittel zur Beeinflussung des Pflanzenwachstums eingesetzt werden. Ihre Wirkung ist besser als die bekannter Wachstumsregulatoren. Die Wirkung zeigt sich sowohl bei Monokotylen, z. B. Getreide, wie Weizen, Gerste, Roggen, Hafer und Reis oder Mais als auch bei Dikotylen (z. B. Sonnenblumen, Tomaten, Soja, Reben, Baumwolle und Raps) und verschiedenen Zierpflanzen, wie Chrysanthemen, Poinsettien und Hibiskus.

Die erfindungsgemäßen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwekken ; sie sollen in jedem Fall eine feine und gleichmäßige Verteilung oder wirksamen Substanz gewährleisten. Die Formulierungen werden in bekannter Weise hergestellt, z. B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage : Lösungsmittel wie Aromaten (z. B. Xylol, Benzol), chlorierte Aromaten (z. B. Chlorbenzole), Paraffine (z. B. Erdölfraktionen), Alkohole (z. B. Methanol, Butanol), Amine (z. B. Ethanolamin, Dimethylformamid) und Wasser ; Trägerstoffe wie natürliche Gesteinsmehle, z. B. Kaoline, Tonerden, Talkum, Kreide und synthetische Gesteinsmehle (z. B. hochdisperse Kieselsäure, Silikate) ; Emulgiermittel, wie nichtionogene und anionische Emulgatoren (z. B. Polyoxyethylen — Fettalkohol — Ether, Alkylsulfonate und Arylsulfonate) und Dispergiermittel, wie Lignin, Sulfitablaugen und Methylcellulose.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet.

Beispiele für solche Zubereitungen sind :

I. Man vermischt 90 Gewichtsteile der Verbindung des Beispiels 1 mit 10 Gewichtsteilen N-Methyl-α-pyrrolidon und erhält eine Lösung, die zur Anwendung in Form kleinster Tropfen geeignet ist.

II. 20 Gewichtsteile der Verbindung des Beispiels 2 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch

Ausgießen und feines Verteilen der Lösung in 100 000 Gewichtsteilen Wasser erhält man eine wäßrige Dispersion, die 0,02 Gew.% des Wirkstoffs enthält.

III. 20 Gewichtsteile der Verbindung des Beispiels 3 werden in einer Mischung gelöst, die aus 40 Gewichtsteilen Cyclohexanon, 30 Gewichtsteilen Isobutanol, 20 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

IV. 20 Gewichtsteile der Verbindung des Beispiels 5 werden in einer Mischung gelöst, die aus 25 Gewichtsteilen Cyclohexanol, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

V. 20 Gewichtsteile der Verbindung des Beispiels 6 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfitablauge und 60 Gewichsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.

VI. 3 Gewichtsteile der Verbindung des Beispiels 1 werden mit 97 Gewichtsteilen feinteiligem Kaolin innig vermischt. Man erhält auf diese Weise ein Stäubemittel, das 3 Gew.% des Wirkstoffs enthält.

VII. 30 Gewichtsteile der Verbindung des Beispiels 2 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gewichtsteile der Verbindung des Beispiels 3 werden mit 10 Teilen Natriumsalz eines Phenolsulfonsäure-harnstoff-formaldehyd-Kondensates, 2 Teilen Kieselgel und 48 Teilen Wasser innig vermischt. Man erhält eine stabile wäßrige Dispersion. Durch Verdünnen mit 100 000 Gewichtsteilen Wasser erhält man eine wäßrige Dispersion, die 0,0 Gew.% Wirkstoff enthält.

IX. 20 Teile der Verbindung des Beispiels 4 werden mit 2 Teilen Calciumsalz der Dodecylbenzolsulfonsäure, 8 Teilen Fettalkohol-polyglykolether, 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z. B. Herbiziden, Insektiziden, Wachstumsregulatoren und Fungiziden, oder auch mit Düngemitteln vermischt und ausgebracht werden.

Fungizide, die mit den erfindungsgemäßen Verbindungen kombiniert werden können, sind beispielsweise :

Schwefel,
Dithiocarbamate und deren Derivate, wie Ferridimethyldithiocarbamat, Zinkdimethyldithiocarbamat, Manganethylenbisdithiocarbamat, Mangan-Zink-ethylendiamin-bis-dithiocarbamat
oder Zinkethylenbisdithiocarbamat,
Tetramethylthiuramidsulfide,
Ammoniak-Komplex von Zink-(N,N-ethylen-bis-dithiocarbamat)
und N,N'-Polyethylen-bis-(thiocarbamoyl)-disulfid,
Zink-(N,N'-propylen-bis-dithiocarbamat),
Ammoniak-Komplex von Zink-(N,N'-propylen-bis-dithiocarbamat)
und N,N'-Propylen-bis(thiocarbamoyl)-disulfid ;

Nitroderivate, wie
Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat ;

heterocyclische Substanzen, wie
N-Trichlormethylthio-tetrahydrophthalimid,
N-(1,1,2,2-Tetrachlorethylthio)-tetrahydrophthalimid,
N-Trichlormethylthio-phthalimid,

2-Heptadecyl-2-imidazolin-acetat,
2,4-Dichlor-6-(o-chloranilino)-s-triazin,
0,0-Diethyl-phthalimidophosphonothioat,
5-Amino-1-(bis-(dimethylamino)-phosphinyl)-3-phenyl-1,2,4-triazol,
5-Ethoxy-3-trichlormethyl-1,2,4-thiadiazol,
2,3-Dicyano-1,4-dithioanthrachinon,
2-Thio-1,3-dithio-(4,5-b)-chinoxalin,
1-(Butylcarbamoyl)-2-benzimidazol-carbaminsäuremethylester,
2-Methoxycarbonylamino-benzimidazol,
2-Rhodanmethylthio-benzthiazol,
4-(2-Chlorphenylhydrazono)-3-methyl-5-isoxazolon,
Pyridin-2-thio-1-oxid,
8-Hydroxychinolin bzw. dessen Kupfersalz,
2,3-Dihydro-5-carboxanilido-6-methyl-1,4-oxathiin-4,4-dioxid,
2,3-Dihydro-5-carboxanilido-6-methyl-1,4-oxathiin,
2-(Furyl-(2))-benzimidazol,
Piperazin-1,4-diyl-bis-(1-(2,2,2-trichlor-ethyl)-formamid),
2-(Thiazolyl-(4))-benzimidazol,
5-Butyl-2-dimethylamino-4-hydroxy-6-methyl-pyrimidin,
Bis-(p-Chlorphenyl)-pyridinmethanol,
1,2-Bis-(3-ethoxycarbonyl-2-thioureido)-benzol,
1,2-Bis-(3-methoxycarbonyl-2-thioureido)-benzol

und weitere Substanzen, wie
Dodecylguanidinacetat,
3-(3-(3,5-Dimethyl-2-oxycyclohexyl)-2-hydroxyethyl)-glutarimid,
Hexachlorbenzol,
N-Dichlorfluormethylthio-N',N'-dimethyl-N-phenyl-schwefelsäurediamid,
2,5-Dimethyl-furan-3-carbonsäureanilid,
2,5-Dimethyl-furan-3-carbonsäure-cyclohexylamid,
2-Cyan-N-(ethylaminocarbonyl)-2-(methoxyimino)-acetamid,
2-Methyl-benzoesäure-anilid,
2-Iod-benzoesäure-anilid,
1-(3,4-Dichloranilino)-1-formylamino-2,2,2-trichlorethan,
2,6-Dimethyl-N-tridecyl-morpholin bzw. dessen Salze,
2,6-Dimethyl-N-cyclododecyl-morpholin bzw. dessen Salze,

DL-Methyl-N-(2,6-dimethyl-phenyl)-N-furoyl(2)-alaninat,
DL-N-(2,6-Dimethyl-phenyl)-N-(2'-methoxyacetyl)-alanin-methylester,
5-Nitro-isophthalsäure-di-isopropylester,
1-(1',2',4'-Triazolyl-1')-(4'-chlorphenoxy)-3,3-dimethylbutan-2-on,
1-(1',2',4'-Triazolyl-1')-1-(4'-chlorphenoxy)-3,3-dimethylbutan-2-ol,
N-(2,6-Dimethylphenyl)-N-chloracetyl-D,L-2-aminobutyrolacton,
N-(n-Propyl)-N-(2,4,6-trichlorphenoxyethyl)-N'-imidazolylharnstoff.

Im nachfolgenden Beispiel wird die Wirkung der erfindungsgemäßen Verbindungen als Pflanzenwachstumsregulatoren dargestellt :

## Beispiel A

Zur Bestimmung der wachstumsregulierungen Eigenschaft der Prüfsubstanzen wurden Testpflanzen auf ausreichend mit Nährstoffen versorgtem Kultursubstrat in Kunststoffgefäßen von ca. 12,5 cm Durchmesser angezogen.

Im Nachauflaufverfahren wurden die zu prüfenden Substanzen in wäßriger Aufbereitung auf die Pflanzen gesprüht. Die beobachtete wachstumsregulierende Wirkung wurde bei Versuchsende durch Wuchshöhenmessung belegt. Die so gewonnenen Meßwerte wurden zur Wuchshöhe der unbehandelten Pflanzen in Relation gesetzt. Als Vergleichssubstanz diente CCC :

$$CH_3-\overset{CH_3}{\underset{CH_3}{\overset{+|}{\underset{|}{N}}}}-CH_2-CH_2-Cl \qquad Cl^-$$

Gleichlaufend zur Reduzierung des Längenwachstums stieg die Farbintensität der Blätter an. Der erhöhte Chlorophyllgehalt läßt eine ebenfalls erhöhte Photosyntheserate und damit eine erhöhte

Ertragsbildung erwarten.
Die Einzeldaten sind der folgenden Tabelle zu entnehmen :
Soja, Sorte SRF 450
Nachauflaufbehandlung, Versuchsdauer : 27 Tage

| Wirkstoff des Beispiels | Aufwandmenge mg/Gefäß | Wuchshöhe % |
|---|---|---|
| - | - | 100 |
| CCC | 1,5 | 99,7 |
| | 6,0 | 96,4 |
| 13 | 1,5 | 50,3 |
| | 6,0 | 48,7 |
| 27 | 1,5 | 45,5 |
| | 6,0 | 39,0 |
| 29 | 1,5 | 58,1 |
| | 6,0 | 53,2 |

**Ansprüche**

1. α-Azolylglykolderivate der allgemeinen Formel I

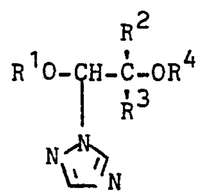

(I)

in welcher
$R^1$ $C_{1-4}$-Alkyl,
$R^2$ $C_{1-6}$-Alkyl oder gegebenenfalls durch Halogen substituiertes Phenyl,
$R^3$ Wasserstoff oder $C_{1-6}$-Alkyl,
$R^4$ Alkyl oder Alkenyl mit bis zu 10 C-Atomen oder halogensubstituiertes Benzyl
bedeuten, sowie deren für Pflanzen verträgliche Salze und Metallkomplexe.

2. Pflanzenwachstumsregulierendes Mittel, enthaltend mindestens eine Verbindung gemäß Anspruch 1.

3. Verfahren zur Regulierung des Pflanzenwachstums, dadurch gekennzeichnet, daß man mindestens eine Verbindung gemäß Anspruch 1 auf die Pflanzen oder deren Lebensraum einwirken läßt.

4. Verfahren zur Herstellung von α-Azolylglykolderivaten der Formel I gemäß Anspruch 1, dadurch gekennzeichnet, daß man

a) einen Alkohol der Formel II

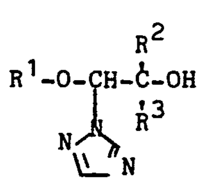

(II)

in der $R^1$, $R^2$, $R^3$ und X die im Anspruch 1 angegebene Bedeutung haben oder ihre Alkali- oder quartären Ammoniumsalze mit einer Verbindung der Formel III

$$R^4\text{—}L \qquad \text{(III)}$$

worin $R^4$ die oben angegebene Bedeutung hat und L für eine nucleophil verdrängbare Abgangsgruppe steht, gegebenenfalls in Gegenwart eines Lösungs- oder Verdünnungsmittels und/oder anorganischer

oder organischer Basen sowie gegebenenfalls unter Zusatz eines Reaktionsbeschleunigers bei Temperaturen zwischen 0 und 120 °C umsetzt oder

b) Acetale der Formel IV

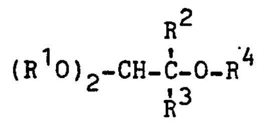
(IV)

in der $R^1$, $R^2$, $R^3$ und $R^4$ die im Anspruch 1 angegebenen Bedeutungen haben, zunächst mit Acetylchlorid oder -bromid und anschließend mit 1, 2, 4-Triazol oder dessen Alkali- oder Erdalkalisalz gegebenenfalls in Gegenwart eines Lösungs- oder Verdünnungsmittels und/oder anorganischer oder organischer Basen bei Temperaturen zwischen 0 und 100 °C umsetzt.

## Claims

1. α-Azolyl-glycol derivatives of the general formula I

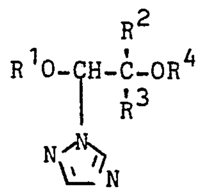
(I)

where
$R^1$ is alkyl of 1 to 4 carbon atoms,
$R^2$ is alkyl of 1 to 6 carbon atoms or phenyl optionally substituted by halogen,
$R^3$ is hydrogen or alkyl of 1 to 6 carbon atoms,
$R^4$ is alkyl or alkenyl of up to 10 carbon atoms, or benzyl substituted by halogen,
and their salts and metal complexes tolerated plants.

2. An agent for regulating plant growth, containing at least one compound as claimed in claim 1.

3. A process for regulating plant growth, wherein at least one compound as claimed in claim 1 is allowed to act on the plants or their habitat.

4. A process for the manufacture of α-azolyl-glycol derivatives of the formula I as claimed in claim 1, wherein an alcohol of the formula II

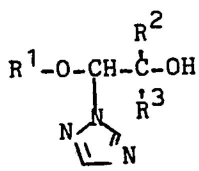
(II)

where $R^1$, $R^2$, $R^3$ and X have the meanings given in claim 1, or an alkali metal salt or quaternary ammonium salt thereof, is reacted with a compound of the formula III

$$R^4—L \quad (III)$$

where $R^4$ has the above meaning and L is a nucleophilically displaceable leaving group, in the presence or absence of a solvent or diluent and/or of an inorganic or organic base and in the presence or absence of a reaction accelerator, at from 0 to 120 °C, or an acetal of the formula IV

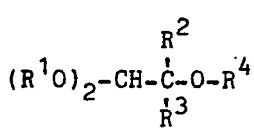
(IV)

where $R^1$, $R^2$, $R^3$ and $R^4$ have the meanings given in claim 1, is reacted first with acetyl chloride or acetyl bromide and then with 1, 2, 4-triazole or an alkali metal salt or alkaline earth metal salt thereof, in the presence or absence of a solvent or diluent and/or of an inorganic or organic base, at from 0 to 100 °C.

**Revendications**

1. Dérivés d'α-azolylglycols de formule générale I

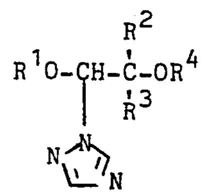

(I)

dans laquelle
$R^1$ représente un alkyle en $C_{1-4}$,
$R^2$ un alkyle en $C_{1-6}$ ou un phényle, éventuellement substitué par un halogène,
$R^3$ l'hydrogène ou un alkyle en $C_{1-6}$,
$R^4$ un alkyle ou alcényle ayant jusqu'à 10 atomes C ou benzyle substitué par halogène
ainsi que leurs sels et complexes métalliques compatibles avec les plantes.

2. Agent de régularisation de la croissance des plantes, contenant au moins un composé selon la revendication 1.

3. Procédé de régularisation de la croissance des plantes, caractérisé par le fait qu'on fait agir au moins un composé selon la revendication 1 sur les plantes ou leur biotope.

4. Procédé de préparation de dérivés d'α-azolylglycols de formule I selon la revendication 1, caractérisé par le fait que

a) on fait agir un alcool de formule II

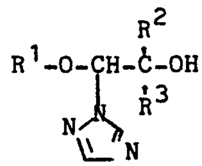

(II)

dans laquelle $R^1$, $R^2$, $R^3$ et X ont la signification donnée dans la revendication 1, ou leurs sels alcalins ou d'ammonium quaternaire, avec un composé de formule III

$$R^4—L \quad (III)$$

dans laquelle $R^4$ a la signification donnée plus haut et L est un groupe de départ déplaçable, nucléophile, éventuellement en présence d'un solvant ou diluant et/ou de bases inorganiques ou organiques ainsi qu'éventuellement avec addition d'un accélérateur de réaction, à des températures comprises entre 0 et 120°, ou

b) on fait agir des acétals de formule IV

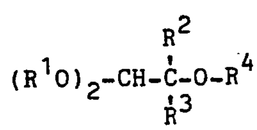

(IV)

dans laquelle $R^1$, $R^2$, $R^3$ et $R^4$ ont les significations données en revendication 1, tout d'abord avec du chlorure ou bromure d'acétyle et ensuite avec du 1, 2, 4-triazole ou son sel alcalin ou alcalino-terreux, éventuellement en présence d'un solvant ou diluant et/ou de bases inorganiques ou organiques, à des températures comprises entre 0 et 100 °C.